# EUROPÄISCHE PATENTSCHRIFT

(11) **EP 0 525 537 B1**
(45) Veröffentlichungstag und Bekanntmachung des Hinweises auf die Patenterteilung: **21.09.1994**
(21) Anmeldenummer: 92112201.6
(22) Anmeldetag: 17.07.1992
(51) Int. Cl.: A61K 31/44

(54) **Verwendung von 1,4-Dihydropyridinen zur Herstellung eines Arzneimittels, zur Behandlung von Neuropathien, die durch Diabetes mellitus verursacht sind**
Use of 1,4-dihydropyridine derivatives for the preparation of a medicament for the treatment of neuropathies caused by diabetes mellitus
Utilisations de dérivés de 1,4-dihydropyridines pour la fabrication d'un médicament pour le traitement de neuropathies provoquées par diabetes mellitus

(30) Priorität: 30.07.1991 DE 4125116
(43) Veröffentlichungstag der Anmeldung: 03.02.1993
(73) Patentinhaber: Troponwerke GmbH & Co. KG, D-51063 Köln (DE)
(72) Erfinder: Traber, Jörg, Dr., W-5204 Lohmar 21 (DE); Gispen, Willem Hendrik, Prof. Dr., Bilthoven (NL)
(74) Vertreter: Dänner, Klaus, Dr.

(56) Entgegenhaltungen:
- NEUROSCIENCE RESEARCH COMMUNICATIONS Bd. 10, Nr. 2, 1992, Seiten 95 - 104; A.C. KAPELLE ET AL.: 'The Ca-antagonist nimodipine counteracts the onset of anexperimental neuropathy in streptozotocin induced diabetic rats'
- PHARMAZEUTISCHE ZEITUNG WISSENSCHAFT Bd. 135, Nr. 1-3, 1990, Seiten 29 - 34; M.WELSCH ET AL.: 'Nimodipin wirkt neuroprotektiv ohne Steigerung der lokalen
- zerebralen Durchblutung'
- NEUROSCIENCE LETTERS Bd. 83, 1987, Seiten 143 - 148; C. VAN DER ZEE ET AL.:'Oral administration of nimodipine accelerates functional recovery followingperipheral nerve damage in the rat'
- J. AUTON. PHARMAC. Bd. 11, Nr. 2, April 1991, Seiten 64 - 71; P. LONGHURST: 'Invitro contractile responses of vasa deferentia from spontaneously diabetic BBrats'
- Science Band 261, Seiten 86-90, 2. Juli 1993
- Diabetologia Band 35, Seiten 1-5, 1992

## Beschreibung

Die Erfindung betrifft die Verwendung von 1,4-Dihydropyridinen mit calciumantagonistischer Wirkung, insbesondere von Nimodipin, zur Herstellung von Arzneimitteln zur Bekämpfung von Schädigungen peripherer Nerven die durch Diabetes verursacht sind.

Die erfindungsgemäß einsetzbaren Dihydropyridine, ihre Herstellung und ihre Verwendung als Kreislauf- und zerebral wirksamer Mittel sind bekannt (vgl. DPS 21 17 571, EP-B-4650). Für den Wirkstoff Nimodipin (1,4-Dihydro-2,6-dimethyl-4-(3'-nitrophenyl)pyridin-3-β-methoxyethylester-5-isopropylester) sind neben den zerebralen Wirkungen wie Verbesserungen des Gedächtnisses, des Lernverhaltens und der Motorik auch bestimmte neuroprotektive Wirkungen bekannt (vgl. Steen P.A. et al, Anesthesiology, 62, 406-411 (1985)). Neben diesen zerebralen Wirkungen des Nimodipins sind auch Wirkungen auf peripheren Nerven beschrieben. In der Publikation von R.E. Sporel-Özakat et al, Nimodipine and central nervous system function: New Vistas, Schattauer Verlag Stuttgart, 71-85 (1989) wird beschrieben, daß nach Verabreichung von Nimodipin an Ratten die peripheren Nerven besser geschützt sind insbesondere bezüglich cis-Platin Neuropathie. In dieser Publikation wird auch darauf hingewiesen, daß der Wirkungsmechanismus für Nimodipin nicht bekannt sei und daß weitere diesbezügliche Untersuchungen dringend erforderlich seien.

In der Publikation von C.E.E.M. van der Zee et al., Neuroscience Letters, 83 (1987) 143-148 wird die positive Wirkung von Nimodipin auf mechanisch geschädigte periphere Nerven beschrieben. Der Stand der Technik enthält keinen Hinweis darauf, daß 1,4-Dihydropyridine mit calciumantagonistischer Wirkung, insbesondere Nimodipin, bei der Therapie und Prophylaxe von Schädigungen der peripheren Nerven, die durch Diabetes verursacht werden, eingesetzt werden kann.

Einer der häufigsten Spätschäden bei Diabetes mellitus ist die periphere Neuropathie. Die Prävalenzrate hierfür liegt bei über 60 % der an Diabetes mellitus erkrankten Patienten. Zu den Symptomen zählen Schmerzen in den Extremitäten, Muskelschwäche bis hin zu Lähmungserscheinungen sowie diverse Dysfunktionen des autonomen Nervensystems wie Diarrhoe oder Impotenz. Durch diese durch Diabetes verursachten Schädigungen wird die Nervenleitgeschwindigkeit sowohl sensorischer als auch motorischer Nerven in Konsistenz verringert. Diese Nervenleitgeschwindigkeit ist somit ein gut meßbarer Parameter zur Bestimmung dieser Schädigungen.

Es gibt bisher keine etablierte Methode zur Behandlung solcher Neuropathien und somit existiert ein dringender Bedarf nach einem therapeutisch wirksamen Mittel.

Es ist bereits versucht worden, Aldosereduktase-Inhibitoren für diese Therapie einzusetzen. Diese Substanzen hemmen das Enzym, welches die Umwandlung von Glukose in Sorbit katalysiert. Bei Diabetes mellitus liegt ein solches Glukose-Überangebot vor, woraus ein Überschuß an Sorbit entsteht, welches z.B. zu Trübungen der Augenlinse führen kann und welches auch als eine Ursache für die Neuropathie, die durch Diabetes verursacht wird, angesehen wird. Aldose-Reduktase-Inhibitoren wurden bereits klinisch in dieser Indikation getestet. Die in sie gesetzten Erwartungen haben sich aber nicht erfüllt.

Wie bereits oben angegeben, ist es auch für den Calciumantagonisten Nimodipin bekannt, daß er bestimmte neuroprotektive Wirkungen besitzt. Er beschleunigt z.B. die Regeneration der Funktion peripherer Nerven nach mechanischer Läsion. Außerdem ist beschrieben, daß das Nimodipin bei der Ratte nach einer speziellen Intoxikation mit dem Cytostatikum cis-Platin selektive Störungen der Nervenleitgeschwindigkeit sensorischer peripherer Nerven antagonisiert. Die Nervenleitgeschwindigkeit motorischer peripherer Nerven wird durch cis-Platin nicht betroffen.

Eine entsprechende Intoxikation mit Acrylamid führt ebenfalls zu einer Reduktion der Leitgeschwindigkeit von sensorischen und motorischen Nerven. Es ist bekannt, daß Nimodipin diese Schädigung peripherer Nerven nicht antagonisiert (vgl. R.E. Sporel-Özakat, Dissertation, Universität Utrecht, 1990, Seiten 93-98). Diese Befunde zeigen, daß die Nervenschutzwirkung von Nimodipin nicht von allgemeiner Art ist und somit nicht aus dem Stand der Technik abgeleitet werden kann. Der Fachmann konnte nicht erwarten, daß Nimodipin eine therapeutische Wirkung auf die spezielle periphere Neuropathie, die durch Diabetes mellitus induziert wird, hat.

Überraschenderweise wurde nun gefunden, daß Nimodipin Schädigungen sensorischer und motorischer peripherer Nerven antagonisiert, die durch chronische Überzuckerung verursacht werden. Dieser Effekt tritt sowohl nach prophylaktischer Verabreichung als auch nach therapeutischer Gabe ein. Damit wird der Fachmann erstmals in die Lage versetzt, periphere Neuropathien, die durch Diabetes mellitus verursacht werden, therapeutisch zu behandeln. Es war für den Fachmann nicht vorhersehbar, daß Calciumantagonisten vom Typ des Nimodipin solche speziellen therapeutischen Wirkungen auf periphere Nerven besitzen.

Die Herstellung des Wirkstoffs Nimodipin und die Herstellung von Arzneimitteln enthaltend Nimodipin zur Behandlung von solchen Neuropathien erfolgt nach üblichen Methoden z.B. durch Verstrecken des Wirkstoffs mit Lösungsmitteln und/oder Trägerstoffen und anschließende Überführung in übliche Formulierungen wie Tabletten, Dragees, Granulate, Sirupe, Emulsionen, Suspensionen, Lösungen unter Verwendung inerter, nicht-toxischer pharmazeutisch geeigneter Hilfsstoffe.

Die Applikation erfolgt in üblicher Weise, vorzugsweise oral. Bei oraler Applikation beträgt die Dosierung etwa 0,05 bis 20 mg/kg, vorzugsweise 0,1 bis 10 mg/kg Körpergewicht.

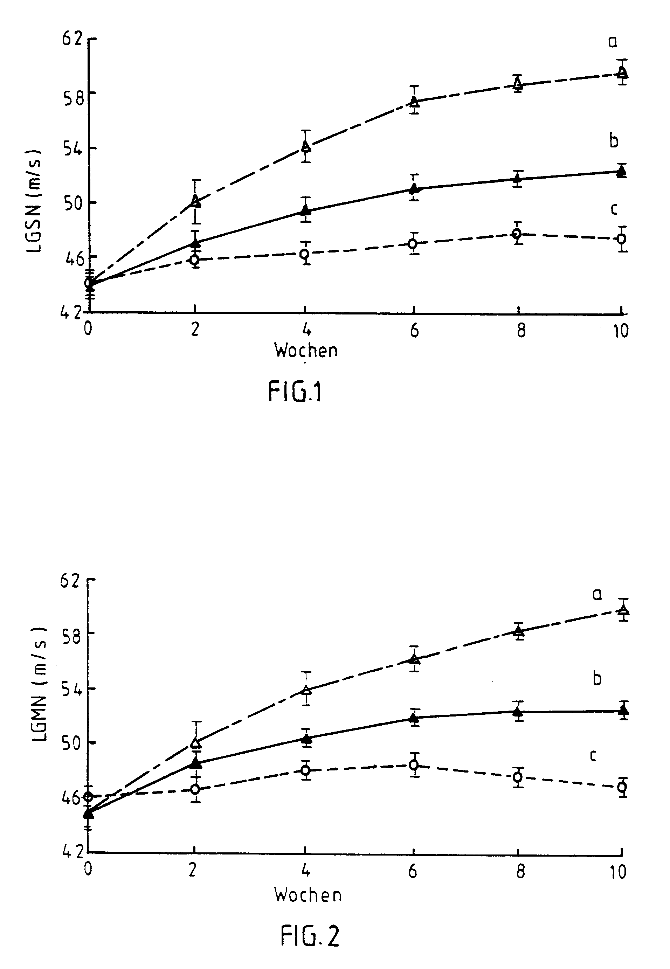

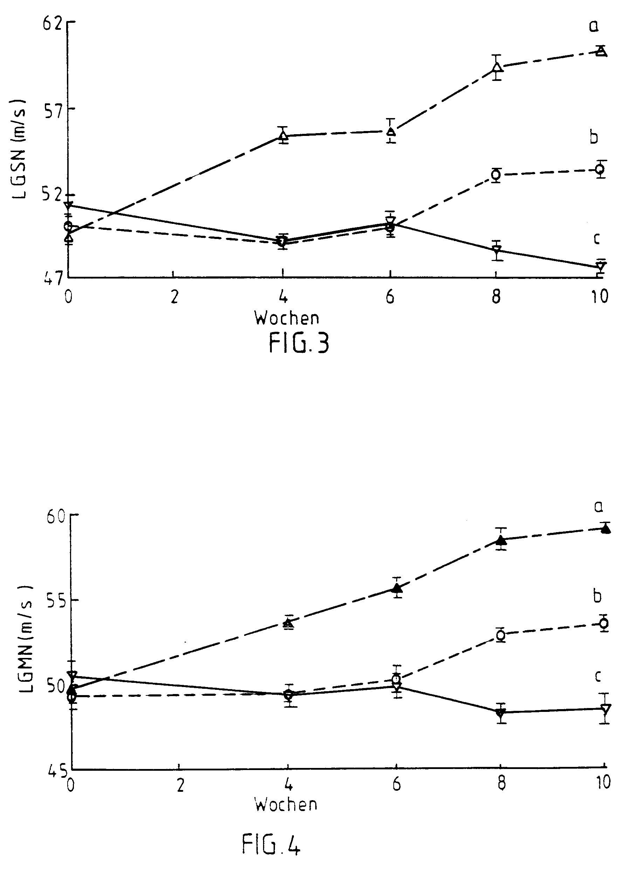

Der überraschende therapeutische Effekt der vorliegenden Erfindung wird durch folgende Anwendungsbeispiele belegt:
Bei männlichen Wistar-Ratten werden die Insulin produzierenden Pankreaszellen geschädigt durch eine einmalige Gabe von Streptozotocin (50 mg/kg i.v.), was zu chronisch erhöhten Glukosespiegeln führt. Bei den so geschädigten diabetischen Ratten sind die Nervleitgeschwindigkeiten deutlich reduziert (vgl. A.K. Sharma et al, Diabetic neuropathy (1987), 237-252 und Y. Harati, Ann. Intern. Med, 107 (1987) 546-559). Die Ergebnisse des Tests sind aus den Kurven der Fig. 1 bis 4 zu entnehmen, Die Kurven a geben jeweils die Nervleitgeschwindigkeit der Kontrollgruppen, die Kurven c diejenige bei geschädigten diabetischen Ratten, die kein Nimodipin erhalten haben und die Kurven b die Nervleitgeschwindigkeit bei diabetischen mit Nimodipin behandelten Ratten wieder, Während bei den gesunden Kontrolltieren die Nervleitgeschwindigkeit sowohl sensorischer (Fig. 1 und 3, Kurven a) als auch motorischer Nerven (Fig. 2 und 4, Kurven a) über die Zeit stetig ansteigt, verändert sich die Nervleitgeschwindigkeit bei den nicht mit Nimodipin behandelten diabetischen Tieren praktisch nicht (jeweils Kurven c für sensorische Nerven in Fig. 1 und 3, für motorische Nerven in Fig. 2 und 4).

Nach Applikation von Nimodipin (20 mg/kg i.p. alle 48 Stunden) wird die durch die Überzuckerung induzierte Störung signifikant ausgeglichen. Dies gilt sowohl für prophylaktische Nimodipinbehandlung (Kurven b in Fig. 1 und 2) als auch für therapeutische Nimodipinbehandlung (Kurven b in Fig. 3 und 4).
- Fig. 1:: Prophylaktischer Effekt von Nimodipin auf die Leitgeschwindigkeit sensorischer Nerven
Es sind jeweils die Mittelwerte (± SEM) für die Leitgeschwindigkeiten sensorischer Nerven (LGSN) 0, 2, 4, 6, 8 und 10 Wochen nach Streptozotocin-Gabe (50 mg/kg i.v.) gezeigt. Gruppe 1 (Kurve b,n=11) wurde mit Nimodipin (20 mg/kg i.p. alle 48 h in 1 ml/kg Polyethylenglykol) behandelt, Gruppe 2 (Kurve c,n=11) nur mit dem Lösungsmittel (1 ml/kg i.p.). Gruppe 3 (Kurve a,n=11) ist eine weder mit Streptozotocin noch mit Nimodipin, sondern nur mit 1 ml/kg Polyethylenglykol behandelte nicht diabetische Kontrollgruppe. Alle Versuche wurden an männlichen Wistar-Ratten durchgeführt (11 - 12 Wochen alt zu Beginn des Experimentes).

Die statistische Analyse ergab, daß bei Streptozotocinbehandelten diabetischen Tieren die Nimodipin-Behandlung zu einer signifikanten Erhöhung der LGSN führte (p<0,001). Die LGSN unbehandelter nicht diabetischer Kontrolltiere lag signifikant höher als die der Lösungsmittel- bzw. Nimodipin-behandelten diabetischen Ratten (p<0,001).
- Fig. 2:: Prophylaktischer Effekt von Nimodipin auf die Leitgeschwindigkeit motorischer Nerven
Es sind jeweils die Mittelwerte (± SEM) für die Leitgeschwindigkeiten motorischer Nerven (LGMN) 0, 2, 4, 6, 8 und 10 Wochen nach Streptozotocin-Gabe (50 mg/kg i.v.) gezeigt. Gruppe 1 (Kurve b,n=11) wurde mit Nimodipin (20 mg/kg i.p. alle 48 h in 1 ml/kg Polyethylenglykol) behandelt, Gruppe 2 (Kurve c,n=11) nur mit dem Lösungsmittel (1 ml/kg i.p.). Gruppe 3 (Kurve a,n=11) ist eine weder mit Streptozotocin noch mit Nimodipin, sondern nur mit 1 ml/kg Polyethylenglykol behandelte nicht diabetische Kontrollgruppe. Alle Versuche wurden an männlichen Wistar-Ratten durchgeführt (11 - 12 Wochen alt zu Beginn des Experimentes).

Die statistische Analyse ergab, daß bei Streptozotocinbehandelten diabetischen Tieren die Nimodipin-Behandlung zu einer signifikanten Erhöhung der LGMN führte (p<0,001). Die LGMN unbehandelter nicht diabetischer Kontrolltiere lag signifikant höher als die der Lösungsmittel- bzw. Nimodipin-behandelten diabetischen Ratten (p<0,001).
- Fig. 3:: Therapeutischer Effekt von Nimodipin auf die Leitgeschwindigkeit sensorischer Nerven
Es sind jeweils die Mittelwerte (± SEM) für die Leitgeschwindigkeiten sensorischer Nerven (LGSN) 0, 4, 6, 8 und 10 Wochen nach Streptozotocin-Gabe (50 mg/kg i.v.) gezeigt. Gruppe 1 (Kurve b,n=12) wurde mit Nimodipin (20 mg/kg i.p. alle 48 h in 1 ml/kg Polyethylenglykol) behandelt, Gruppe 2 (Kurve c,n=12) nur mit dem Lösungsmittel (1 ml/kg i.p.). Die Behandlung der diabetischen Ratten mit Nimodipin bzw. Lösungsmittel erfolgte 4 Wochen nach Beginn der Streptozotocin-Gabe, einem Zeitpunkt, bei dem ein signifikanter Abfall der LGSN im Vergleich zu nicht mit Streptozotocin behandelten, nicht diabetischen Kontrolltieren meßbar war (Gruppe 3, Kurve a,n=12). Alle Versuche wurden an männlichen Wistar-Ratten durchgeführt (11 - 12 Wochen alt zu Beginn des Experimentes).

Die statistische Analyse ergab, daß bei Streptozotocinbehandelten diabetischen Tieren die Nimodipin-Behandlung zu einer signifikanten Erhöhung der LGSN führte (p<0,001). Die LGSN unbehandelter nicht diabetischer Kontrolltiere lag signifikant höher als die der Lösungsmittel- bzw. Nimodipin-behandelten diabetischen Ratten (p<0,001).
- Fig. 4:: Therapeutischer Effekt von Nimodipin auf die Leitgeschwindigkeit motorischer Nerven
Es sind jeweils die Mittelwerte (± SEM) für die Leitgeschwindigkeiten motorischer Nerven (LGMN) 0, 4, 6, 8 und 10 Wochen nach Streptozotocin-Gabe (50 mg/kg i.v.) gezeigt. Gruppe 1 (Kurve b,n=12) wurde mit Nimodipin (20 mg/kg i.p. alle 48 h in 1 ml/kg Polyethylenglykol) behandelt, Gruppe 2 (Kurve c,n=12) nur mit dem Lösungsmittel (1 ml/kg i.p.). Die Behandlung der diabetischen Ratten mit Nimodipin bzw. Lösungsmittel erfolgte 4 Wochen nach Beginn der Streptozotocin-Gabe, einem Zeitpunkt, bei dem ein signifikanter Abfall der LGMN im Vergleich zu nicht mit Streptozotocin behandelten, nicht diabetischen Kontrolltieren meßbar war (Gruppe 3, Kurve a,n=12). Alle Versuche wurden an männlichen Wistar-Ratten durchgeführt (11 - 12 Wochen alt zu Beginn des Experimentes).

Die statistische Analyse ergab, daß bei Streptozotocinbehandelten diabetischen Tieren die Nimodipin-Behandlung zu einer signifikanten Erhöhung der LGMN führte (p<0,001). Die LGMN unbehandelter nicht diabetischer Kontrolltiere lag signifikant höher als die der Lösungsmittel- bzw. Nimodipin-behandelten diabetischen Ratten (p<0,001).

## Patentansprüche

1. Verwendung von Calciumantagonisten aus der Familie der 1,4-Dihydropyridine bei der Herstellung von Arzneimitteln zur Bekämpfung von Neuropathien, die durch Diabetes verursacht sind.

2. Verwendung gemäß Anspruch 1, dadurch gekennzeichnet, daß Nimodipin als Wirkstoff eingesetzt wird.

## Claims

1. Use of calcium antagonists from the 1,4-dihydropyridine family in the preparation of medicaments for controlling neuropathies which are caused by diabetes.

2. Use according to Claim 1, characterized in that nimodipine is employed as the active compound.

## Revendications

1. Utilisation d'antagonistes du calcium de la famille des 1,4-dihydropyridines dans la préparation de médicaments pour lutter contre des neuropathies qui sont provoquées par le diabète.

2. Utilisation selon la revendication 1, caractérisée en ce qu'on met en oeuvre la nimodipine comme substance active.
